# EUROPEAN PATENT SPECIFICATION

(11) **EP 0 489 756 B1**
(45) Date of publication and mention of the grant of the patent: **13.12.1995**
(21) Application number: 90911888.7
(22) Date of filing: 16.08.1990
(51) Int. Cl.: C07D 317/42

(54) **PROCESS FOR THE PREPARATION OF HALOGENATED 2,2-BIS(TRIFLUOROMETHYL)-1,3-DIOXOLANES**
VERFAHREN ZUR HERSTELLUNG HALOGENIERTER 2,2-BIS(TRIFLUORMETHYL)-1,3-DIOXOLANE
PROCEDE POUR LA PREPARATION DE 2,2-BIS(TRIFLUOROMETHYLE)-1,3-DIOXOLANES HALOGENES

(30) Priority: 28.08.1989 US 399100
(43) Date of publication of application: 17.06.1992
(73) Proprietor: E.I. DU PONT DE NEMOURS AND COMPANY, Wilmington Delaware 19898 (US)
(72) Inventor: MANZER, Leo, E., Wilmington, DE 19803 (US); RESNICK, Paul, R., Wilmington, DE 19805 (US)
(74) Representative: Jones, Alan John
(86) International application number: US9004505
(87) International publication number: WO9103472

(56) References cited:
- EP-A- 0 317 981
- DE-A- 3 603 103
- DE-A- 3 842 986
- GB-A- 1 361 346
- US-A- 4 535 175
- J. Fluorine Chem., Vol. 9, no. 9, 1977, p. 359-375, R.D. Bagnall et al: "New inhalation anaesthetics: I. fluorinated 1,3-dioxolane derivatives", see especially pages 368-374

## Description

### Background of the Invention

The invention concerns a catalytic process for the preparation of halogenated 2,2-bis(trifluoromethyl)-1,3-dioxolanes.

2,2-Bis(trifluoromethyl)-1,3-dioxolane is a known compound which may be halogenated according to a number of known methods to produce various halo-isomers at the 4,5 or 4 and 5 positions. For example, U.S. Patent No. 2,925,424 describes a batch photochemical chlorination of 2,2-bis(trifluoromethyl)-1,3-dioxolane. The reaction was conducted at 50°C for 2.5 hours. A 68% yield of 2,2-bis(trifluoromethyl)-4,4,5,5-tetrachloro-1,3-dioxolane was obtained upon fractionation of crude product. See Example 9.

U.S. Patent No. 4,535,175 discloses a batch photochemical chlorination of 2,2-bis(trifluoromethyl)-1,3-dioxolane to yield a mixture of di-, tri- and tetrachloro derivatives. The reaction proceeds rather slowly. Example 1 of the patent details the production of 2,2-bis(trifluoromethyl)-4,4,5-trichloro-5-fluoro-1,3-dioxolane by reacting 2,2-bis(trifluoromethyl)-4,4,5,5,-tetrachloro-1,3-dioxolane, HF and antimony chloride at 70°C for 5 hours.

DE-A 3,842,986, discloses a process for preparing dioxoles by dechlorinating perhalogenated 4,5-dichloro-1,3-dioxolanes in the presence of lithium aluminum hydride and titanium chloride.

EP-A 317,981 discloses a process for preparing 1,1-dichloro-1,2,2,2-tetrafluoroethane by isomerization of 1,1,2-trichloro-1,2,2-trifluoroethane to 1,1,1-trichloro-2,2,2-trifluoroethane followed by fluorination of the latter with HF in the presence of a halide or oxide containing at least one element selected from Al, Cr, Mg, Ca, Sr, Ba, Fe, Ni, Co, Sb, Nb or Mn.

R. D. Bagnall et al., J. Fluorine Chem., 9, 359-375 (1977) disclose the preparation of various fluorinated 1,3-dioxolane derivatives, including 2,2-bis-trifluoromethyl- 1,3-dioxolanes wherein F, Cl or a combination thereof occupy the 4- and 5-positions. Di-, tri- and tetrachloro derivatives are fluorinated with SbF₃Cl₂ or SbF₃-SbCl₅ mixture.

U.S. Patent No. 3,794,791 discloses a batch photochlorination of 2,2-bis(trifluoromethyl)-1,3-dioxolane at -15°C. Example 1 describes the synthesis of 2,2-bis(trifluoromethyl)-4-chloro-1,3-dioxolane. Example 2 of the patent describes the synthesis of 2,2-bis(trifluoromethyl)-4,5-dichloro-1,3-dioxolane.

G.B. 1,361,346 discloses the preparation of 2,2-bis(trifluoromethyl)-4,5-dichloro-4,5-difluoro-1,3-dioxolane by fluorinating 2,2-bis(trifluoromethyl)-4,4,5,5,-tetrachloro-1,3-dioxolane with SbF₃/SbC1₅ at 120°C.

### Summary of the Invention

The invention provides a process for the preparation of 2,2-bis(perhaloalkyl)-4,4,5,5-tetrachloro-1,3-dioxolane by chlorinating 2,2-bis(perhaloalkyl)-1,3-dioxolane with a source of chlorine in the presence of a catalyst which contains at least one of La, Ni, Sn, Zn Co, Fe, or Cu.

The invention also provides a process for the preparation of fluorinated 2,2-bis(perhaloalkyl)-1,3-dioxolanes of the formula 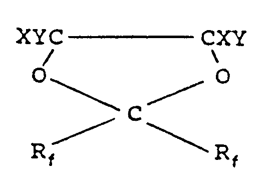
wherein X is Cl, each Y is independently Cl or F and at least one Y is F, and each R_{f} is independently perhaloalkyl in which the alpha carbon is substituted by at least one fluorine atom.

The process comprises fluorinating 2,2-bis(perhaloalkyl)-4,4,5,5-tetrachloro-1,3-dioxolane with a source of fluorine under fluorination conditions, so as to effect fluorine-chlorine exchange, in the presence of a catalyst which is a catalyst as noted above in the chlorination reaction or is chromium (III) oxide, i.e., Cr₂O₃ or one or more of a metal supported on carbon wherein the metals are chosen from Cr, Co, La, Fe, Ni, Cu, Sn, or Zn.

### Detailed Description

Preferably, R_{f} is trifluoromethyl. The 2,2-bis(perhaloalkyl)-1,3-dioxolane starting material for the chlorination is a known compound which may be readily prepared by reacting perfluoroacetone and ethylene chlorohydrin under basic conditions as described in U.S. Patent No. 2,925,424.

The chlorination is preferably conducted in the vapor phase, and is preferably conducted continuously. However, the reaction may be conducted in the liquid phase using the reactants as solvents, at the same temperatures noted for the vapor phase, under autogenous pressure. Preferred temperatures for the reaction are 250° - 300°C. The molar ratio of chlorine to 2,2-bis(perhaloalkyl)-1,3-dioxolane is preferably about 4:1 to 10:1, more preferably about 4:1 to 5:1. The reaction time is generally about 1 to 120 seconds, and is preferably about 30 to 60 seconds. The reaction pressure is preferably about 1 to 20 atmospheres, more preferably about 10 to 20 atmospheres. The preferred source of chlorine is chlorine gas. The catalyst metal may be in the form of any soluble compound of the metal such as the oxide, oxyhalide, halide, pseudohalide, nitrate, sulfate, or organic salt such as acetate and propionate. The halides include chlorides, fluorides and bromides. The pseudohalides include cyanides, cyanates and thiocyanates. The form of the catalyst is not critical and may be pellets, powders or granules. Preferably, the catalysts are used in a fixed bed; however, fluidized bed reactors may also be used. Preferably, the catalysts are in the form of their chlorides, and preferably are supported on carbon. The preferred catalyst for the chlorination is CuCl₂/C.

### General Procedure for Preparing Catalysts MClₓ/C

The desired amount of metal chloride was dissolved in water (35 to 75 mL) and the entire solution poured over 40 g of commercial carbon granules (Girdler 411, 0.32 cm pellets). The resulting mixture was allowed to stand at room temperature for one hour and was then placed in a vacuum oven at 110°C for 16 to 24 hours to remove the water. The catalyst was then pretreated by heating in an atmosphere of nitrogen gas at 400°C followed by heating in HF at 400°C prior to its use as a fluorination catalyst. For chlorination reactions, the catalyst was heated in an atmosphere of nitrogen gas at 400°C followed by adjusting the temperature to the desired value and treatment with chlorine gas. Preferably, 0.1 - 30% b.w. based on the support of active metal is incorporated in the catalyst.

### Catalyst Preparation

The following catalysts were prepared by the general procedure for MClₓ/C:

| | |
|---|---|
| CoCl₂/C | 35 g CoCl₂.6H₂O/35 mL H₂O |
| FeCl₃/C | 39.7 g FeCl₃.6H₂O/35 mL H₂O |
| ZnCl₂/C | 20.44 g ZnCl₂/75 mL H₂O |
| NiCl₂/C | 34.94 g NiCl₂2.6H₂O/35 mL H₂O |
| LaCl₃/C | 62.43 g LaCl3.7H₂I/75 mL H₂O |
| CrCl₃/C | 39.17 g CrCl₃.6H₂O/60 mL H₂O |
| SnCl₂/C | 38.36 g SnCl₂.2H₂O/70 mL H₂O |
| CuCl₂/C | 25.06 g CuCl₂.2H₂O/70 mL H₂O |

Cr₂O₃ is commercially available and was treated with HF as described above prior to its use as a fluorination catalyst.

The catalysts having differing anions are prepared analogously.

The fluorination is preferably conducted in the vapor phase, and is preferably conducted continuously. The reaction may be conducted in the liquid phase as indicated for the chlorination. The reaction temperature is preferably 150° to 350°C, more preferably 150° to 200°C. The preferred source of fluorine is HF. The molar ratio of HF to 2,2-bis(perhaloalkyl)-4,4,5,5-tetrachloro-1,3-dioxolane is preferably about 2:1 to 10:1, more preferably about 2:1 to 5:1. Preferred reaction time is about 1 to 120 seconds, more preferably about 30 to 60 seconds. The preferred catalyst for the fluorination is Cr, more preferably unsupported Cr₂O₃.

The production of the fluoro-compounds may be conducted in two steps, with, e.g., isolation of a 2,2-bis(perhaloalkyl)-4,4,5,5-tetrachloro-1,3-dioxolane intermediate, or may be conducted in one enclosure with the addition of the fluorine source preferably after substantial completion of the first reaction.

The catalysts of the invention are extremely active and selective. In the fluorination, where Cr₂O₃ is the catalyst, the ratio of trans to cis isomers is desirably high.

The products of the process of the invention are useful as intermediates in the production of perhalodioxoles of the formula 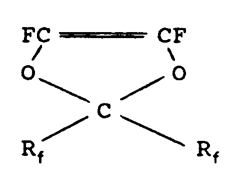
by dehalogenation of the appropriate precursor according to conventional processes, for example, as disclosed in U.S. Patent Nos. 4,535,175, 3,865,845, and 3,978,030.

Preferably, trans-2,2-bis(perhaloalkyl)-4,5-difluoro-4,5-dichloro-1,3-dioxolane is used to produce the dioxole. A multi-plate distillation column may be used to separate the cis and trans isomers. Preferably, the cis isomer as well as 2,2-bis(perhaloalkyl)-4,4,5,5-tetrachloro-1,3-dioxolane and -4-fluoro-4,5,5-trichloro-1,3-dioxolane are recycled to the fluorination step.

The dioxole in turn may be used to prepare homopolymers and copolymers which possess advantageous properties such as chemical inertness to hydrogen fluoride, optical clarity and film-forming ability.

For example, the dioxoles may be copolymerized under standard polymerization conditions with tetrafluoroethylene to form crystalline copolymers suitable for use as a dielectric in electronic equipment. Preferably, in these applications the dioxole is employed in an amount of about 12 mole % or less.

Polymers having more than about 12% dioxole are more generally amorphous, and are soluble in various organic liquids, e.g., 1,1,2-trichloro-1,2,2-trifluoroethane. These polymers are useful in the production of chemically inert, stain and weather resistant coatings and finishes.

Further, the dioxoles may be reacted with vinylidene fluoride or tetrafluoroethylene to produce plastic and/or elastomeric terpolymers useful in the production of corrosion-resistant seals, gaskets or linings.

Finally, the dioxoles may be homopolymerized to produce amorphous resins suitable for use as transparent glazing materials and sight glasses in apparatuses employed in handling chemically corrosive materials.

In particular, the amorphous polymers are useful in the production of optical fiber cladding materials, e.g., in accordance with U.S. Patent Nos. 4,530,569 and 4,754,009.

Various fluoropolymers have been proposed from time to time for this purpose, mainly because of their good performance under a variety of temperature and atmospheric conditions and resistance to many chemicals. A good polymeric cladding material for optical fibers should be completely amorphous because crystallites present in polymers would cause light scattering. Further, it should have a high glass transition temperature, Tg, especially if intended for use at high temperatures because above its Tg, it would lose some of its desirable physical properties; and, in particular, it would be unable to maintain good bonding to the fiber core. A desirable Tg would be above 85°C, preferably above 120°C.

As the amount of dioxole in the copolymer increases, the Tg also increases, although not necessarily in a linear fraction.

A homopolymer of dioxole appears to be amorphous and has a high Tg. However, dioxole is a much more expensive monomer than tetrafluoroethylene so that use of dioxole homopolymers, rather than of dioxole/tetrafluoroethylene copolymers, is economically much less attractive. Furthermore, the copolymers are easier to fabricate. The dipolymers have low refractive indices, which is a particularly desirable feature for optical fiber cladding. Furthermore, films of these copolymers are clear and transparent, compared with hazy or translucent films of crystalline polymers. For this reason, the amorphous copolymers of the present invention are suitable for such applications as, for example, windows for chemical reactors, especially for processes using or manufacturing hydrogen fluoride.

Amorphous terpolymers can be made by copolymerizing certain ethylenically unsaturated monomers with perfluoro-2,2-dimethyl-1,3-dioxole and tetrafluoroethylene. These include selected olefins, vinyl compounds, and perfluoromonomers. Typical olefins are, for example, ethylene, propylene, 1-butene, isobutylene, trifluoropropene, and trifluoroethylene. Vinyl monomers can be, for example, vinyl fluoride, vinylidene fluoride, and chlorotrifluoroethylene. Perfluoromonomers may be of different chemical types, for example, perfluoropropene, perfluoro(1,3-dioxole), perfluoro(alkyl vinyl ethers), methyl 3-[1-[difluoro(trifluoroethenyl)oxy]methyl]-1,2,2,2-tetrafluoroethoxy]-2,2,3,3-tetrafluoropropanoate 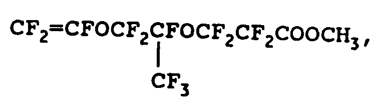
and 2-[1-[difluoro[(trifluoroethenyl)oxy]methyl]-1,2,2,2-tetrafluorooethoxy]-1,1,2,2-tetrafluoroethanesulfonyl fluoride

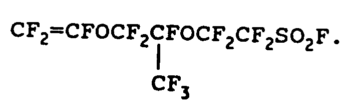

The proportion of dioxole in the amorphous terpolymers of this invention should preferably be at least 12 mole percent of the tetrafluoroethylene content, while the mole percent content of the third monomer should be the smallest of all three monomers. Outside these limits, either an amorphous terpolymer may not be obtained; or, if made, its maximum tensile modulus and strength may not be realized.

Copolymerization is carried out in the presence of a free radical generator, preferably at a slightly elevated temperature, for example, 55°-65°C. Well-agitated pressure equipment should be used.

In addition to tetrafluoroethylene, amorphous copolymers may be fabricated from the dioxole and chlorotrifluoroethylene; vinylidene fluoride; hexafluoropropylene; trifluoroethylene; perfluoro(alkyl vinyl ethers) of the formula CF₂=CFOR_{F}, where R_{F} is a normal perfluoroalkyl radical having 1-3 carbon atoms; fluorovinyl ethers of the formula CF₂=CFOQZ, where Q is a perfluorinated alkylene radical containing 0-5 ether oxygen atoms, wherein the sum of the C and O atoms in Q is 2 to 10, and Z is a group selected from the class consisting of -CN, -COF, and -OCH₃, where R is a C_{l-C4} alkyl; vinyl fluoride; and (perfluoroalkyl)ethylene, R_{*f*}CH=CH₂, where R_{*f*} is a C_{l-C8} normal perfluoroalkyl radical.

The maximum preferred mole percentage of the comonomer in the copolymers are as follows:
for tetrafluoroethylene, 35;
for chlorotrifluoroethylene, 30
for vinylidene fluoride, 20;
for hexafluoropropylene, 5;
for trifluoroethylene, 30;
for CF₂=CFOR_{F}, 30;
for CF₂=CFOQZ, 20;
for vinyl fluoride, 25; and
for R_{*f*}CH=CH₂, 10.

Without further elaboration, it is believed that one skilled in the art can, using the preceding description, utilize the present invention to its fullest extent. The following preferred specific embodiments are, therefore, to be construed as merely illustrative and not limitative of the remainder of the disclosure in any way whatsoever.

In the preceding text and the following examples, all temperatures are set forth uncorrected in degrees Celsius and all parts and percentages are by weight, unless otherwise indicated. Amounts of compounds listed in the Tables are given in area %. C.T. represents contact time.

In the following examples, the numbers identifying the compounds in the tables are as follows:
D456 - 2,2-Bis(trifluoromethyl)-1,3-dioxolane
D436 - 2,2-Bis(trifluoromethyl)-4,5-dichloro-1,3-dioxolane
D436a - 2,2-Bis(trifluoromethyl)-4,4-dichloro-1,3-dioxolane
D426 - 2,2-Bis(trifluoromethyl)-4,4,5-trichloro-1,3-dioxolane
D416 - 2,2-Bis(trifluoromethyl)-4,4,5,5-tetrachloro-1,3-dioxolane
D417 - 2,2-Bis(trifluoromethyl)-4-fluoro-4,5,5-trichloro-1,3-dioxolane
D418a - 2,2-Bis(trifluoromethyl)-4,4-difluoro-5,5-dichloro-1,3-dioxolane
D418 - 2,2-Bis(trifluoromethyl)-4,5-dichloro-4,5-difluoro-1,3-dioxolane
D419 - 2,2-Bis(trifluoromethyl)-4,4,5-trifluoro-5-chloro-1,3-dioxolane.

### EXAMPLES

### General Procedure for Chlorination

The reactor (0.5 inch ID Inconel\/nickel alloy pipe) was charged with the designated amount of catalyst, sealed and placed into a sand bath. The bath was heated to 400°C, at which time nitrogen gas at a flow rate of 50 ml/minute was passed through the reactor to remove all traces of water. Chlorine gas at a flow rate of 25.7 cc/min was passed through the reactor and the temperature adjusted to the desired value. The flow of nitrogen gas was turned off and the flow of D456 started. The flow of chlorine and D456 were adjusted to give the desired molar ratio.

The reactor effluent was scrubbed with aqueous potassium hydroxide to remove Cl₂, HCl and HF and sampled on line by a HP 5890 gas chromatograph using a 5 foot Krytox\ perfluorinated polyether column. Conditions were 70°C for 3 minutes followed by temperature programming to 180°C at a rate of 6°C/minute. Helium flow was 35 cc/minute.

### General Procedure for Fluorination

The reactor (0.5 inch ID Inconel\ nickel alloy pipe) was charged with the designated amount of catalyst, sealed and placed into a sand bath. The bath was heated to 400°C, at which time nitrogen gas at a flow rate of 50 ml/minute was passed through the reactor to remove all traces of water. The temperature was lowered to 200°C, and HF and nitrogen gas (1:4 molar ratio) was passed through the reactor, and the nitrogen flow was decreased with time until neat HF was being passed through the reactor. At this point, the temperature is raised to 350°C and maintained there for 15 to 30 minutes. The temperature is then decreased to the desired temperature, and the D416 flow is started. The flow of HF and D416 were adjusted to give the desired molar ratio.

The reactor effluent was scrubbed with aqueous potassium hydroxide to remove Cl₂, HCl and HF and sampled on line by a HP 5890 gas chromatograph using a 5 foot Krytox\ perfluorinated polyether column. Conditions were 70°C for 3 minutes followed by temperature programming to 180°C at a rate of 6°C/minute. Helium flow was 35 cc/minute.

### EXAMPLES 1 - 16

### Chlorination of 2,2-Bis(trifluoromethyl)-1,3-Dioxolane (D456)

| CuCl₂/C 19.7 grams (30 cc) | | | | | | | |
|---|---|---|---|---|---|---|---|
| Exp | Temp | Cl₂/D456 | C.T. | D436 | D426 | D416 | Unk |
| 1 | 225 | 6/1 | 30 | 14 | 40 | 43 | 3 |
| 2 | 235 | 6/1 | 30 | 5 | 29 | 62 | 3 |
| 3 | 245 | 6/1 | 30 | 3 | 21 | 74 | 2 |
| 4 | 255 | 6/1 | 30 | 1 | 9 | 88 | 2 |
| 5 | 265 | 6/1 | 30 | 0 | 5 | 93 | 2 |
| 6 | 195 | 6/1 | 60 | 43 | 33 | 16 | 8 |
| 7 | 205 | 6/1 | 60 | 25 | 44 | 24 | 5 |
| 8 | 215 | 6/1 | 60 | 10 | 44 | 42 | 4 |
| 9 | 225 | 6/1 | 60 | 5 | 34 | 57 | 3 |
| 10 | 235 | 6/1 | 60 | 1 | 18 | 80 | 1 |
| 11 | 245 | 6/1 | 60 | 0 | 7 | 90 | 3 |
| 12 | 255 | 6/1 | 60 | 0 | 2 | 95 | 3 |
| 13 | 265 | 6/1 | 60 | 0 | 0 | 97 | 3 |
| 14 | 275 | 6/1 | 60 | 0 | 0 | 96 | 4 |
| 15 | 285 | 6/1 | 60 | 0 | 0 | 95 | 5 |
| 16 | 285 | 4/1 | 60 | 0 | 1 | 95 | 4 |

The conversion of D456 to products was 100%, as shown in the Table.

### EXAMPLES 17-23

### Chlorination of 2,2-Bis(trifluoromethyl)-1,3-Dioxolane (D456)

| CuCl₂/C 19.7 grams (30 cc) | | | | | | | |
|---|---|---|---|---|---|---|---|
| Exp | Temp | Cl₂/D456 | C.T. | D426 | D416 | D417 | Unk |
| 17 | 265 | 6/1 | 60 | 1 | 97 | 0 | 2 |
| 18 | 265 | 6/1 | 60 | 1 | 97 | 0 | 1 |
| 19 | 265 | 6/1 | 60 | 1 | 97 | 0 | 3 |
| 20 | 265 | 5/1 | 75 | 1 | 97 | 1 | 0 |
| 21 | 265 | 4/1 | 75 | 3 | 95 | 0 | 2 |
| 22 | 265 | 5/1 | 60 | 2 | 95 | 0 | 3 |
| 23 | 275 | 5/1 | 60 | 0 | 97 | 0 | 2 |

The conversion of D456 to products was 100%, as shown in the Table.

### EXAMPLES 24-28

### Chlorination of 2,2-Bis(trifluoromethyl)-1,3-Dioxolane (D456)

| LaCl₃/C 4.25 grams (5 cc) | | | | | | | | | |
|---|---|---|---|---|---|---|---|---|---|
| Exp | Temp | Cl₂/D456 | C.T. | D456 | D436 | D426 | D416 | D417 | Unk |
| 24 | 200 | 6/1 | 10 | 3 | 74 | 12 | 10 | 0 | 1 |
| 25 | 225 | 6/1 | 10 | 4 | 64 | 11 | 18 | 0 | 3 |
| 26 | 250 | 6/1 | 10 | 1 | 43 | 9 | 43 | 1 | 2 |
| 27 | 275 | 6/1 | 10 | 0 | 22 | 6 | 67 | 1 | 4 |
| 28 | 300 | 6/1 | 10 | 0 | 9 | 3 | 72 | 3 | 13 |

### EXAMPLES 29-32

### Chlorination of 2,2-Bis(trifluoromethyl)-1,3-Dioxolane (D456)

| SnCl₂/C 4.0 grams (5 cc) | | | | | | | | |
|---|---|---|---|---|---|---|---|---|
| Exp | Temp | Cl₂/D456 | C.T. | D436 | D426 | D416 | D417 | Unk |
| 29 | 225 | 6/1 | 10 | 36 | 37 | 23 | 0 | 3 |
| 30 | 250 | 6/1 | 10 | 13 | 29 | 53 | 1 | 4 |
| 31 | 275 | 6/1 | 10 | 2 | 8 | 74 | 2 | 9 |
| 32 | 275 | 5/1 | 10 | 2 | 9 | 70 | 2 | 12 |

The conversion of D456 to products was 100%, as shown in the Table.

### EXAMPLES 33-37

### Chlorination of 2,2-Bis(trifluoromethyl)-1,3-Dioxolane (D456)

| FeCl₃/C 3.4 grams (5 cc) | | | | | | | | | |
|---|---|---|---|---|---|---|---|---|---|
| Exp | Temp | Cl₂/D456 | C.T. | D456 | D436 | D426 | D416 | D417 | Unk |
| 33 | 200 | 6/1 | 10 | 11 | 36 | 2 | 8 | 0 | 43 |
| 34 | 150 | 6/1 | 10 | 36 | 51 | 1 | 0 | 0 | 12 |
| 35 | 200 | 6/1 | 1 | 67 | 21 | 1 | 1 | 1 | 9 |
| 36 | 250 | 6/1 | 1 | 37 | 25 | 1 | 6 | 0 | 31 |
| 37 | 250 | 4/1 | 1 | 42 | 19 | 1 | 5 | 0 | 33 |

### EXAMPLES 38-41

### Chlorination of 2,2-Bis(trifluoromethyl)-1,3-Dioxolane (D456)

| ZnCl₂/C 3.8 grams (5 cc) | | | | | | | | | |
|---|---|---|---|---|---|---|---|---|---|
| Exp | Temp | Cl₂/D456 | C.T. | D456 | D436 | D426 | D416 | D417 | Unk |
| 38 | 200 | 6/1 | 10 | 27 | 62 | 5 | 4 | 0 | 2 |
| 39 | 225 | 6/1 | 10 | 15 | 70 | 10 | 4 | 0 | 1 |
| 40 | 250 | 6/1 | 10 | 7 | 66 | 15 | 11 | 0 | 1 |
| 41 | 275 | 6/1 | 10 | 1 | 28 | 13 | 50 | 2 | 6 |

### EXAMPLES 42-51

### Fluorination of 2,2-Bis(trifluoromethyl)-4,4,5,5-tetrachloro-1,3-Dioxolane

| Cr₂O₃ 28.9 g (30 cc) -12 + 20 mesh | | | | | | | | | | |
|---|---|---|---|---|---|---|---|---|---|---|
| Exp | Temp | HF/D416/HCl | C.T. | D419 | tD418 | cD418 | D418a | D417 | D416 | Unk |
| 42 | 175 | 2/1/0 | 60 | 2 | 74 | 18 | 0 | 4 | 0 | 1 |
| 43 | 200 | 2/1/0 | 60 | 37 | 10 | 3 | 36 | 10 | 1 | 0 |
| 44 | 165 | 2/1/0 | 60 | 19 | 37 | 9 | 13 | 20 | 1 | 1 |
| 45 | 175 | 2/1/0 | 60 | 15 | 62 | 16 | 2 | 5 | 0 | 0 |
| 46 | 175 | 3/1/0 | 45 | 3 | 77 | 19 | 0 | 0 | 0 | 1 |
| 47 | 175 | 3/1/4 | 45 | 13 | 66 | 18 | 1 | 2 | 0 | 0 |
| 48 | 175 | 4/1/4 | 40 | 4 | 75 | 20 | 0 | 2 | 0 | 0 |
| 49 | 175 | 5/1/4 | 35 | 3 | 75 | 20 | 0 | 1 | 0 | 1 |
| 50 | 175 | 5/1/4 | 40 | 3 | 75 | 20 | 0 | 1 | 0 | 1 |
| 51 | 175 | 5/1/4 | 40 | 4 | 74 | 20 | 0 | 1 | 0 | 1 |
| t = trans c = cis | | | | | | | | | | |

### EXAMPLES 52-54

### Fluorination of 2,2-Bis-trifluoromethyl)-4,4,5,5-tetrachloro-1,3-Dioxolane

| CoCl₂/C 12.3 g (30 cc) | | | | | | | |
|---|---|---|---|---|---|---|---|
| Exp | Temp | HF/D416 | C.T. | tD418 | cD418 | D417 | Unk |
| 52 | 200 | 10/1/0 | 30 | 44 | 35 | 8 | 7 |
| 53 | 200 | 5/1/0 | 60 | 39 | 33 | 16 | 6 |
| 54 | 165 | 10/1/0 | 30 | 47 | 37 | 8 | 3 |

### EXAMPLES 55-59

### Fluorination of 2,2-Bis(trifluoromethyl)-4,4,5,5-tetrachloro-1,3-Dioxolane

| Cr₂O₃ 14.4 g (15 cc) -12 + 20 mesh | | | | | | | | |
|---|---|---|---|---|---|---|---|---|
| Exp | Temp | HF/D416 | C.T. | D419 | tD418 | cD418 | D417 | Unk |
| 55 | 200 | 3/1/0 | 30 | 1 | 72 | 22 | 0 | 5 |
| 56 | 200 | 3/1/0 | 30 | 0 | 71 | 26 | 0 | 3 |
| 57 | 210 | 3/1/0 | 30 | 1 | 73 | 24 | 0 | 2 |
| 58 | 220 | 3/1/0 | 30 | 1 | 72 | 24 | 0 | 3 |
| 59 | 230 | 3/1/0 | 30 | 1 | 73 | 23 | 1 | 2 |

### COMPARATIVE EXAMPLES 60-68

### Fluorination of 2,2-Bis(trifluoromethyl)-4,4,5,5,-tetrachloro-1,3-Dioxolane

| LaCl₃/C 4.25 g (5 cc) | | | | | | | | |
|---|---|---|---|---|---|---|---|---|
| Exp | Temp | HF/D416 | C.T. | tD418 | cD418 | D417 | D416 | Unk |
| 60 | 225 | 2/1/0 | 10 | 2 | 4 | 85 | 3 | 6 |
| 61 | 250 | 2/1/0 | 10 | 14 | 13 | 67 | 0 | 5 |
| 62 | 275 | 2/1/0 | 10 | 26 | 27 | 0 | 0 | 4 |
| 63 | 300 | 2/1/0 | 10 | 34 | 33 | 26 | 0 | 7 |
| 64 | 300 | 2/1/0 | 20 | 43 | 35 | 11 | 0 | 10 |
| 65 | 300 | 2/1/0 | 20 | 44 | 36 | 9 | 0 | 11 |
| 66 | 250 | 2/1/0 | 40 | 41 | 38 | 14 | 0 | 7 |
| 67 | 250 | 2/1/0 | 40 | 38 | 35 | 20 | 0 | 7 |
| 68 | 300 | 2/1/0 | 40 | 46 | 31 | 4 | 0 | 11 |

### EXAMPLES 69-72

### Fluorination of 2,2-Bis(trifluoromethyl)-4,4,5,5-tetrachloro-1,3-Dioxolane

| FeCl₃/C 3.4 g (5 cc) | | | | | | | | |
|---|---|---|---|---|---|---|---|---|
| Exp | Temp | HF/D416 | C.T. | tD418 | cD418 | D417 | D416 | Unk |
| 69 | 200 | 2/1/0 | 5 | 0 | 0 | 24 | 71 | 5 |
| 70 | 225 | 2/1/0 | 5 | 0 | 0 | 42 | 54 | 8 |
| 71 | 275 | 2/1/0 | 10 | 7 | 8 | 76 | 1 | 8 |
| 72 | 275 | 3/1/0 | 10 | 9 | 10 | 70 | 0 | 11 |

### EXAMPLES 73-79

### Fluorination of 2,2-Bis(trifluoromethyl)-4,4,5,5-tetrachloro-1,3-Dioxolane

| NiCl₂/C 1.66 g (3 cc) 1/8-inch Pellets | | | | | | | | |
|---|---|---|---|---|---|---|---|---|
| Exp | Temp | HF/D416 | C.T. | tD418 | cD418 | D417 | D416 | Unk |
| 73 | 200 | 2/1/0 | 5 | 0 | 0 | 5 | 91 | 4 |
| 74 | 220 | 2/1/0 | 5 | 0 | 0 | 7 | 88 | 5 |
| 75 | 240 | 2/1/0 | 5 | 0 | 0 | 13 | 80 | 7 |
| 76 | 260 | 2/1/0 | 5 | 0 | 0 | 31 | 61 | 6 |
| 77 | 280 | 2/1/0 | 5 | 1 | 2 | 45 | 39 | 10 |
| 78 | 280 | 4/1/0 | 5 | 5 | 4 | 66 | 10 | 13 |
| 79 | 325 | 4/1/0 | 5 | 8 | 7 | 34 | 2 | 48 |

### EXAMPLES 80-83

### Fluorination of 2,2-Bis(trifluoromethyl)-4,4,5,5-tetrachloro-1,3-Dioxolane

| CuCl₂/C 19.7 g (30 cc) | | | | | | | | |
|---|---|---|---|---|---|---|---|---|
| Exp | Temp | HF/D416 | C.T. | tD418 | cD418 | D417 | D416 | Unk |
| 80 | 200 | 3/1/0 | 30 | 2 | 2 | 66 | 13 | 16 |
| 81 | 200 | 3/1/0 | 30 | 6 | 5 | 73 | 3 | 13 |
| 82 | 250 | 3/1/0 | 30 | 4 | 4 | 85 | 0 | 5 |
| 83 | 250 | 3/1/0 | 30 | 4 | 4 | 85 | 0 | 6 |

### EXAMPLES 84-85

### Fluorination of 2,2-Bis(trifluoromethyl)-4,4,5,5-tetrachloro-1,3-Dioxolane

| SnCl₂/C 4.0 g (5 cc) | | | | | | | | |
|---|---|---|---|---|---|---|---|---|
| Exp | Temp | HF/D416 | C.T. | tD418 | cD418 | D417 | D416 | Unk |
| 84 | 275 | 2/1/0 | 10 | 9 | 10 | 74 | 2 | 5 |
| 85 | 275 | 2/1/0 | 20 | 19 | 17 | 60 | 0 | 4 |

### EXAMPLES 86-87

### Fluorination of 2,2-Bis(trifluoromethyl)-4,4,5,5-tetrachloro-1,3-Dioxolane

| ZnCl₂/C 3.8 g (5 cc) | | | | | | | | |
|---|---|---|---|---|---|---|---|---|
| Exp | Temp | HF/D416 | C.T. | tD418 | cD418 | D417 | D416 | Unk |
| 86 | 300 | 2/1/0 | 10 | 6 | 7 | 73 | 10 | 4 |
| 87 | 250 | 2/1/0 | 10 | 1 | 2 | 53 | 44 | 1 |

### EXAMPLE 88

### Examples Describing the Rearrangement of Cis-D418 to Trans-D418

A mixture of 150 g D-418 (65.5% trans isomer), 6.0 g antimony pentachloride, and 20 g anhydrous hydrogen fluoride was heated at 100° for one hour and 135° for five hours. The reaction mixture was added to ice/ice water and the lower layer washed with 400 ml ice water to give 139.3 g of product. Analysis showed this product to contain 1.3 g D-419 and 138.0 g D-418 (79.8% trans isomer). Thus the amount of trans D-418 rose from 98.3 g to 110.1 g, while the amount of cis isomer fell from 51.7 to 27.9 g.

A mixture of 40.5 g D-418 (53.5% trans isomer), 4.0 g antimony pentachloride, and 20 g anhydrous hydrogen fluoride was heated at 100° for one hour and 135° for four hours. The reaction mixture was added to ice/ice water and the lower layer separated to give 35.4 g of product containing 26.7 g trans D-418 and 8.5 g cis D-418 (78.4% trans isomer). Thus the amount of trans D-418 rose from 21.7 g to 26.7 g, while the amount of cis D-418 declined from 18.8 g to 8.5. g.

### EXAMPLE 89

### Variation in Yield of Perfluoro-2,2-bis-trifluoromethyl-1,3-dioxole (PDD) with cis/trans bis-2,2-trifluoromethyl-4,5-dichloro-4,5-difluoro-1,3-dioxolane (D418)

A mixture of 24.9 g magnesium turnings and 395 ml tetrahydrofuran was heated 60°. After addition of 2.75 ml, 1,2-dibromoethane approximately 4.5 g bis-2,2-trifluoromethyl-4,5-dichloro-4,5-difluoro-1,3-dioxolane (D418) was added. The reaction mixture was cooled to 35-40° and a total of 133 g cis/trans D418 added. After stirring for 15 minutes, the mixture was distilled until the pot temperature reached 70°. The distillate boiling to 45° was washed with 100 ml of ice water. Gas chromatographic analysis of the lower layer showed it to be greater than 99% PDD.

| Percent Trans Isomer in Starting D418 | Yield of PDD Percent |
|---|---|
| 97.7 | 60.7 |
| 83.6 | 51.7 |
| 64.7 | 40.2 |

Claim 3, step a provides a process for the preparation of 2,2-bis(perhaloalkyl)-4,4,5,5-tetrachloro-1,3-dioxolane by chlorinating 2,2-bis(perhaloalkyl)-1,3-dioxolane with a source of chlorine in the presence of a catalyst which contains at least one of La, Ni, Sn, Zn, Co, Fe, or Cu.

The step b also provides a process for the preparation of fluorinated 2,2-bis(perhaloalkyl)-1,3-dioxolanes of the formula 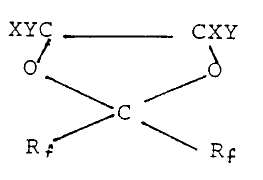
wherein X is C1, each Y is independently C1 or F and at least one Y is F, and each R_{f} is independently perhaloalkyl in which the alpha carbon is substituted by at least one fluorine atom.

Claim 4 includes the steps of claim 3 and also fluorinating 2,2-bis(perhaloalkyl)-4,4,5,5-tetrachloro-1,3-dioxolane with a source of fluorine under fluorination conditions, so as to effect fluorine-chlorine exchange, in the presence of a catalyst which is a catalyst as noted above in the chlorination reaction or is chromium (III) oxide, i.e., Cr₂0₃ or one or more of a metal supported on carbon wherein the metals are chosen from Cr, Co, La, Fe, Ni, Cu, Sn, or Zn.

The catalyst of claim 1 is further defined in claim 5 as a metal chloride. The catalyst metal may be in the form of any soluble compound of the metal such as the oxide, oxyhalide, halide, pseudohalide, nitrate, sulfate, or organic salt such as acetate and propionate. Preferably, the catalysts are in the form of their chlorides, and preferably, as stated in claim 6, the catalysts are supported on carbon. The preferred catalyst for the chlorination is CuC1_{2/}C.

Claims 14, 15 and 19 provide a process for the preparation of 2,2-bis(perhaloalkyl)-4,4,5,5,5-tetrachloro-1,3-dioxolane by chlorinating 2,2-bis(perhaloalkyl)-1,3-dioxolane with a source of chlorine in the presence of a catalyst which contains at least one of La, Ni, Sn, Zn, Co, Fe, or Cu.

The process comprises fluorinating 2,2-bis(perhaloalkyl)-4,4,5,5-tetrachloro-1,3-dioxolane with a source of fluorine under fluorination conditions to effect fluorine-chlorine exchange, in the presence of a catalyst which is a catalyst as noted above in the chlorination reaction or is chromium (III) oxide, i.e., Cr₂0₃ or one or more of a metal supported on carbon wherein the metals are chosen from Cr, Co, La, Fe, Ni, Cu, Sn, or Zn.

The products of the process for the invention are useful as intermediates in the production or perhalodioxoles of the formula 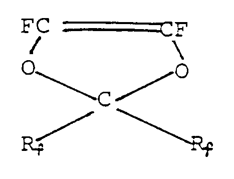
by dehalogenation of the appropriate precursor according to conventional processes, for example, as disclosed in U.S. Patent Nos. 4,535,175, 3,865,845, and 3,978,030.

Preferably, trans-2,2-bis(perhaloalkyl)-4,5-difluoro-4,5-dichloro-1,3-dioxolane is used to produce the dioxole. A multi-plate distillation column may be used to separate the cis and trans isomers. This use of the trans isomer is set forth in claims 16, 17, 18 and 20.

The dioxole in turn may be used to prepare homopolymers and copolymers which possess advantageous properties such as chemical inertness to hydrogen fluoride, optical clarity and film-forming ability.

For example, the dioxoles may be copolymerized under standard polymerization conditions with tetrafluoroethylene to form crystalline copolymers suitable for use as a dielectric in electronic equipment. Preferably, in these applications the dioxole is employed in an amount of about 12 mole % or less.

Polymers having more than about 12% dioxole are more generally amorphous, and are soluble in various organic liquids, e.g., 1,1,2-trichloro-1,2,2-trifluoroethane. These polymers are useful in the production of chemically inert, stain and weather resistant coatings and finishes.

Further, the dioxoles may be reacted with vinylidene fluoride or tetrafluoroethylene to produce plastic and/or elastomeric terpolymers useful in the production of corrosion-resistant seals, gaskets or linings. Homopolymers and copolymers are set forth in claim 21.

Various fluoropolymers as set forth in claim 22 have been proposed from time to time for this purpose, mainly because of their good performance under a variety of temperature and atmospheric conditions and resistance to many chemicals.

## Claims

1. A process for the production of a halogenated 2,2-bis(perhaloalkyl)-1,3-dioxolane of the formula 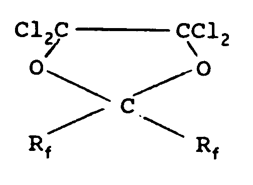 wherein each R_{f} independently is perhaloalkyl in which the alpha carbon atom is substituted by at least one fluorine atom, comprising reacting under chlorination conditions a 2,2-bis(perhaloalkyl)-1,3-dioxolane and a source of chlorine in the presence of a catalyst which is at least one of La, Ni, Sn, Zn, Fe, Co or Cu.

2. A process for the production of a halogenated 2,2-bis(perhaloalkyl)-1,3-dioxolane of the formula 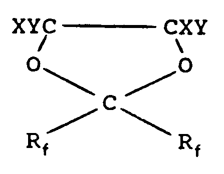 wherein X is Cl and each Y is independently Cl or F and at least one Y is F, and wherein each R_{f} independently is perhaloalkyl in which the alpha carbon atom is substituted by at least one fluorine atom, comprising reacting, under fluorination conditions, 2,2-bis(perhaloalkyl)-4,4,5,5-tetrachloro-1,3-dioxolane and a source of fluorine in the presence of a catalyst which is at least one of Cr, La, Ni, Sn, Zn, Fe, Co or Cu_{.}

3. In a process for the production of a halogenated 2,2-bis(perhaloalkyl)-1,3-dioxolane comprising
(a) chlorinating 2,2-bis(perhaloalkyl)-1,3-dioxolane to produce a compound of the formula 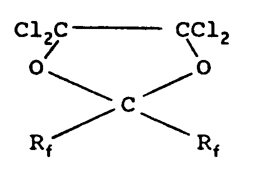 and
(b) fluorinating the product compound of (a) to produce a compound of the formula 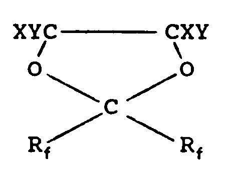 wherein X is Cl and Y is Cl or F, at least one Y being F, and wherein each R_{f} independently is perhaloalkyl in which the alpha carbon atom is substituted by at least one fluorine atom,
the improvement comprising conducting the chlorination in (a) in the presence of a catalyst which is at least one of La, Ni, Sn, Zn, Fe, Co or Cu.

4. In a process for the production of a halogenated 2,2-bis(perhaloalkyl)-1,3-dioxolane comprising
(a) chlorinating 2,2-bis(perhaloalkyl)-1,3-dioxolane, and
(b) fluorinating the product compound of (a) to produce a compound of the formula 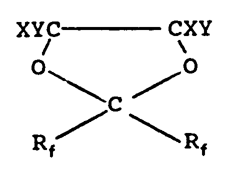 wherein X is Cl and Y is Cl or F, at least one Y being F, and wherein each R_{f} independently is perhaloalkyl in which the alpha carbon atom is substituted by at least one fluorine atom,
the improvement comprising conducing the fluorination in (b) in the presence of a catalyst which is at least one of Cr, La, Ni, Sn, Zn, Fe, Co or Cu.

5. A process according to claim 1, wherein the catalyst is in the form of a supported metal chloride.

6. A process according to claim 5, wherein the catalyst is in the form of metal chloride supported on carbon.

7. A process according to claim 2, wherein the catalyst is in the form of a supported metal chloride.

8. A process according to claim 2, wherein the catalyst is Cr₂O₃.

9. A process according to claim 3, wherein the catalyst in (b) is Cr₂O₃.

10. A process according to claim 1, wherein the catalyst is CuCl₂/C.

11. A process according to claim 1, wherein the temperature is about 250-300°C, the ratio of chlorine to 2,2-bis(perhaloalkyl)-1,3-dioxolane is about 4:1-10:1, the reaction time is about 1-120 sec, and the reaction pressure is about 1-20 atm.

12. A process according to claim 1, wherein R_{f} is CF₃.

13. A process according to claim 2, wherein R_{f} is CF₃.

14. In a process for the production of 2,2-bis(perhaloalkyl)-1,3-dioxole of the formula 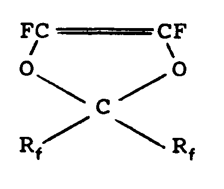 wherein each R_{f} independently is perhaloalkyl in which the alpha carbon atom is substituted by at least one fluorine atom, comprising chlorinating a corresponding 2,2-bis(perhaloalkyl)-1,3-dioxolane, fluorinating the product and subsequently dehalogenating to form the 1,3-dioxole,
the improvement comprising conducting the chlorination in the presence of a catalyst which is at least one of La, Ni, Sn, Zn Fe, Co or Cu.

15. A process according to claim 14, wherein the improvement further comprises conducting the fluorination in the presence of a catalyst which is at least one of Cr, La, Ni, Sn, Zn, Fe, Co or Cu.

16. A process according to claim 14, wherein the improvement further comprises, prior to dehalogenating, separating trans-2,2-bis(perhaloalkyl)-4,5-difluoro-4,5-dichloro-1,3-dioxolane and dehalogenating substantially only said isomer.

17. A process according to claim 15, wherein the improvement further comprises, prior to dehalogenating, separating trans-2,2-bis(perhaloalkyl)-4,5-difluoro-4,5-dichloro-1,3-dioxolane and dehalogenating substantially only said isomer.

18. A process for the production of a 2,2-bis(perhaloalkyl)-1,3-dioxole of the formula 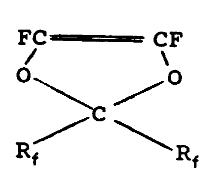 wherein each R_{f} independently is perhaloalkyl in which the alpha carbon atom is substituted by at least one fluorine atom, comprising dehalogenating a corresponding trans-2,2-bis(perhaloalkyl)-4,5-difluoro-4,5-dichloro-1,3-dioxolane which has been separated from a mixture of said trans isomer and other isomers.

19. In a process for the production of 2,2,-bis(perhaloalkyl)-1,3-dioxole of the formula 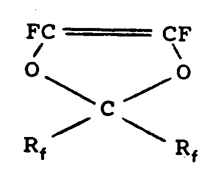 wherein each R_{f} independently is perhaloalkyl in which the alpha carbon atom is substituted by at least one fluorine atom, comprising chlorinating a corresponding 2,2-bis(perhaloalkyl)-1,3-dioxolane, fluorinating the product and subsequently dehalogenating to form the 1,3-dioxole,
the improvement comprises conducting the fluorination in the presence of a catalyst which is at least one of Cr, La, Ni, Sn, Zn, Fe, Co or Cu.

20. In a process for the preparation of a polymer formed from monomers which comprise 2,2-bis(perhaloalkyl)-1,3-dioxoles of the formula 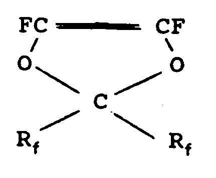 wherein each R_{f} independently is perhaloalkyl in which the alpha carbon atom is substituted by at least one fluorine atom, the improvement comprising preparing the dioxoles by dehalogenating corresponding trans-2,2-bis(perhaloalkyl)-4,5-difluoro-4,5-dichloro-1,3-dioxolanes separated from a mixture of cis and trans isomers.

21. A process according to claim 20, wherein the polymer is produced by homopolymerization of the dioxole, copolymerization of the dioxole with tetrafluoroethylene or copolymerization of the dioxole with vinylidene fluoride.

22. In a process for the preparation of a polymer formed from monomers which comprise 2,2-bis(perhaloalkyl)-1,3-dioxoles of the formula 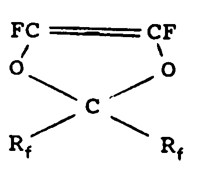 wherein each R_{f} independently is perhaloalkyl in which the alpha carbon atom is substituted by at least one fluorine atom, the improvement comprising preparing the dioxoles by dehalogenating corresponding trans-2,2-bis(perhaloalkyl)-4,5-difluoro-4,5-dichloro-1,3-dioxolanes produced by enhancing the amount of trans-isomer in a mixture of cis- and trans-isomers by rearranging cis- to trans-isomers.

## Patentansprüche

1. Verfahren zur Herstellung eines halogenierten 2,2-Bis(perhalogenalkyl)-1,3-dioxolans der Formel 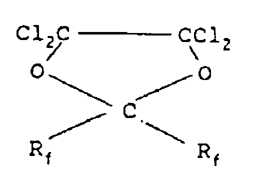 worin R_{f} jeweils unabhängig für Perhalogenalkyl steht, in dem das α-Kohlenstoffatom mit wenigstens einem Fluoratom substituiert ist, welches die Umsetzung unter Fluorierungsbedingungen eines 2,2-Bis(perhalogenalkyl)-1,3-dioxolans und einer Chlorquelle in Gegenwart eines Katalysators, der wenigstens einer aus La, Ni, Sn, Zn, Fe, Co oder Cu ist, umfaßt.

2. Verfahren zur Herstellung eines halogenierten 2,2-Bis(perhalogenalkyl)-1,3-dioxolans der Formel 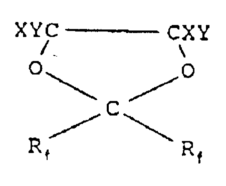 worin X für Cl steht, und Y jeweils unabhängig für Cl oder F steht und wenigstens ein Y für F steht, und worin R_{f} jeweils unabhängig für Perhalogenalkyl steht, in dem das α-Kohlenstoffatom mit wenigstens einem Fluoratom substituiert ist, welches die Umsetzung unter Fluorierungsbedingungen von 2,2-Bis(perhalogenalkyl)-4,4,5,5-tetrachlor-1,3-dioxolan und einer Fluorquelle in Gegenwart eines Katalysators, der wenigstens einer aus Cr, La, Ni, Sn, Zn, Fe, Co oder Cu ist, umfaßt.

3. Bei einem Verfahren zur Herstellung eines halogenierten 2,2-Bis(perhalogenalkyl)-1,3-dioxolans, umfassend
(a) Chlorieren von 2,2-Bis(perhalogenalkyl)-1,3-dioxolan zur Herstellung einer Verbindung der Formel 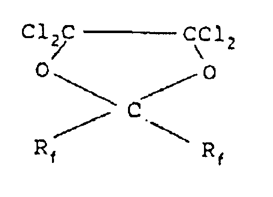 und
(b) Fluorieren der Produktverbindung von (a) zur Herstellung einer Verbindung der Formel 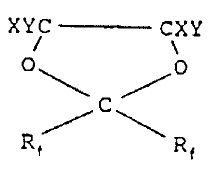 worin X für Cl steht, und Y für Cl oder F steht, wobei wenigstens ein Y für F steht, und worin R_{f} jeweils unabhängig für Perhalogenalkyl steht, in dem das α-Kohlenstoffatom mit wenigstens einem Fluoratom substituiert ist,
die Verbesserung umfaßt, daß die Durchführung der Chlorierung in (a) in Gegenwart eines Katalysators, der wenigstens einer aus La, Ni, Sn, Zn, Fe, Co und Cu ist, erfolgt.

4. Bei einem Verfahren zur Herstellung eines halogenierten 2,2-Bis(perhalogenalkyl)-1,3-dioxolans, umfassend
(a) Chlorieren von 2,2-Bis(perhalogenalkyl)-1,3-dioxolan und
(b) Fluorieren der Produktverbindung von (a) zur Herstellung einer Verbindung der Formel 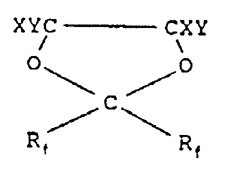 worin X für Cl steht und Y für Cl oder F steht, wobei wenigstens ein Y für F steht, und worin R_{f} jeweils unabhängig für Perhalogenalkyl steht, in dem das α-Kohlenstoffatom mit einem wenigstens einem Fluoratom substituiert ist,
die Verbesserung umfaßt, daß die Durchführung der Chlorierung in (b) in Gegenwart eines Katalysators, der wenigstens einer aus La, Ni, Sn, Zn, Fe, Co und Cu ist, erfolgt.

5. Verfahren nach Anspruch 1, bei dem der Katalysator in Form eines trägergebundenen Metallchlorids vorliegt.

6. Verfahren nach Anspruch 5, bei dem der Katalysator in Form von Metallchlorid auf Kohle als Träger vorliegt.

7. Verfahren nach Anspruch 2, bei dem der Katalysator in Form eines trägergebundenen Metallchlorids vorliegt.

8. Verfahren nach Anspruch 2, bei dem der Katalysator Cr₂O₃ ist.

9. Verfahren nach Anspruch 3, bei dem der Katalysator in (b) Cr₂O₃ ist.

10. Verfahren nach Anspruch 1, bei dem der Katalysator CuCl₂/C ist.

11. Verfahren nach Anspruch 1, bei dem die Temperatur etwa 250 bis 300 °C beträgt, das Verhältnis von Chlor zu 2,2-Bis(perhalogenalkyl)-1,3-dioxolan etwa 4:1 bis 10:1 beträgt, die Reaktionszeit etwa 1 bis 120 sec beträgt und der Reaktionsdruck etwa 1-20 Atmosphären beträgt.

12. Verfahren nach Anspruch 1, bei dem R_{f} CF₃ ist.

13. Verfahren nach Anspruch 2, bei dem R_{f} CF₃ ist.

14. Bei einem Verfahren zur Herstellung von 2,2-Bis(perhalogenalkyl)-1,3-dioxol der Formel 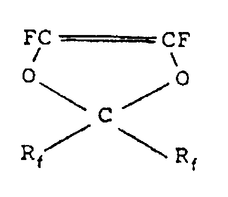 worin R_{f} jeweils unabhängig für Perhalogenalkyl steht, in dem das α-Kohlenstoffatom mit wenigstens einem Fluoratom substituiert ist, das die Chlorierung eines entsprechenden 2,2-Bis(perhalogenalkyl)-1,3-dioxolans, die Fluorierung des Produkts und die anschließende Dehalogenierung unter Bildung des 1,3-Dioxols umfaßt, umfaßt die Verbesserung die Durchführung der Chlorierung in Gegenwart eines Katalysators, der wenigstens einer von La, Ni, Sn, Zn, Fe, Co oder Cu ist.

15. Verfahren nach Anspruch 14, bei dem die Verbesserung außerdem die Durchführung der Fluorierung in Gegenwart eines Katalysators umfaßt, der wenigstens einer von Cr, La, Ni, Sn, Zn, Fe, Co oder Cu ist.

16. Verfahren nach Anspruch 14, bei dem die Verbesserung außerdem vor der Dehalognierung die Abtrennung von trans-2,2-Bis(perhalogenalkyl)-4,5-difluor-4,5-dichlor-1,3-dioxolan und die Dehalogenierung von im wesentlichen nur des Isomeren umfaßt.

17. Verfahren nach Anspruch 15, bei dem die Verbesserung außerdem vor der Dehalogenierung die Abtrennung von trans-2,2-Bis(perhalogenalkyl)-4,5-difluor-4,5-dichlor-1,3-dioxolan und die Dehalogenierung von im wesentlichen nur des Isomeren umfaßt.

18. Verfahren zur Herstellung eines 2,2-Bis(perhalogenalkyl)-1,3-dioxols der Formel 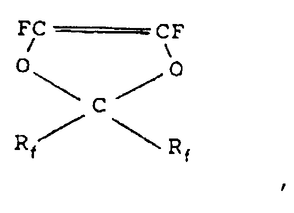 worin R_{f} jeweils unabhängig für Perhalogenalkyl steht, in dem das α-Kohlenstoffatom mit wenigstens einem Fluoratom substituiert ist, umfassend die Dehalogenierung eines entsprechenden trans-2,2-Bis(perhalogenalkyl)-4,5-difluor-4,5-dichlor-1,3-dioxolans, das aus einem Gemisch des trans-Isomeren und weiterer Isomere abgetrennt worden ist.

19. Bei einem Verfahren zur Herstellung von trans-2,2-Bis-(perhalogenalkyl)-1,3-dioxol der Formel 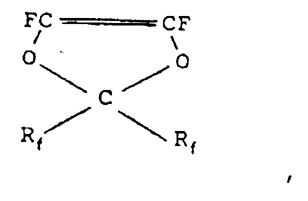 worin R_{f} jeweils unabhängig für Perhalogenalkyl steht, in dem das α-Kohlenstoffatom mit wenigstens einem Fluoratom substituiert ist, das die Chlorierung eines entsprechenden 2,2-Bis(perhalogenalkyl)-1,3-dioxolans, die Fluorierung des Produkts und die anschließende Dehalogenierung unter Bildung des 1,3-Dioxols umfaßt, die Verbesserung der Durchführung der Fluorierung in Gegenwart eines Katalysators, der wenigstens einer von Cr, La, Ni, Sn, Zn, Fe, Co oder Cu ist, umfaßt.

20. Bei einem Verfahren zur Herstellung eines Polymeren, gebildet aus den Monomeren, die 2,2-Bis(perhalogenalkyl)-1,3-dioxole der Formel 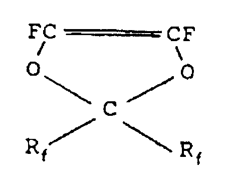 umfassen, worin R_{f} jeweils unabhängig für Perhalogenalkyl steht, in dem das α-Kohlenstoffatom durch wenigstens ein Fluoratom substituiert ist, die Verbesserung der Herstellung der Dioxole durch Dehalogenierung der entsprechenden trans-2,2-Bis(perhalogenalkyl)-4,5-difluor-4,5-dichlor-1,3-dioxolane, die aus einem Gemisch von cis- und trans-Isomeren abgetrennt worden sind, umfaßt.

21. Verfahren nach Anspruch 20, bei dem das Polymer durch Homopolymerisation des Dioxols, Copolymerisation des Dioxols mit Tetrafluorethylen oder Copolymerisation des Dioxols mit Vinylidenfluorid hergestellt worden ist.

22. Bei einem Verfahren zur Herstellung eines Polymeren, gebildet aus den Monomeren, die 2,2-Bis(perhalogenalkyl)-1,3-dioxole der Formel 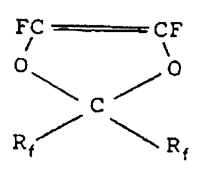 umfassen, worin R_{f} jeweils unabhängig für Perhalogenalkyl steht, in dem das α-Kohlenstoffatom durch wenigstens ein Fluoratom substituiert ist, umfassend die Verbesserung die Herstellung der Dioxole durch Dehalogenierung der entsprechenden trans-2,2-Bis(perhalogenalkyl)-4,5-difluor-4,5-dichlor-1,3-dioxolane, die hergestellt wurden, indem die Menge des trans-Isomeren in einem Gemisch von cis- und trans-Isomeren durch Umlagerung der cis- zu trans-Isomeren vergrößert worden ist.

## Revendications

1. Un procédé pour la production d'un 2,2-bis(perhalogénoalkyl)-1,3-dioxolanne halogéné de la formule 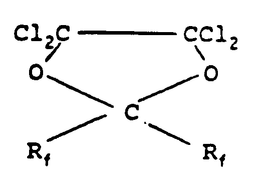 où chaque R_{f} est indépendamment un groupe perhalogénoalkyle dont l'atome de carbone alpha est substitué par au moins un atome de fluor, consistant à faire réagir, dans des conditions de chloration, un 2,2-bis(perhalogénoalkyl)-1,3-dioxolanne et une source de chlore en présence d'un catalyseur qui est au moins l'un de La, Ni, Sn, Zn, Fe, Co ou Cu.

2. Un procédé pour la production d'un 2,2-bis(perhalogénoalkyl)-1,3-dioxolanne halogéné de la formule 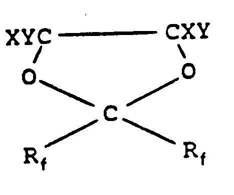 où X est Cl et chaque Y est indépendamment Cl ou F et au moins un Y est F, et où chaque R_{f} est indépendamment un groupe perhalogénoalkyle dont l'atome de carbone alpha est substitué par au moins un atome de fluor, consistant à faire réagir, dans des conditions de fluoration, un 2,2-bis(perhalogénoalkyl)-4,4,5,5-tétrachloro-1,3-dioxolanneet une source de fluor en présence d'un catalyseur qui est au moins l'un de Cr, La, Ni, Sn, Zn, Fe, Co ou Cu.

3. Dans un procédé pour la production d'un 2,2-bis(perhalogénoalkyl)-1,3-dioxolanne halogéné consistant à
(a) chlorer un 2,2-bis(perhalogénoalkyl)-1,3-dioxolanne pour produire un composé de la formule 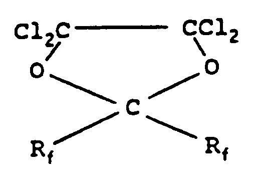 et
(b) fluorer le composé produit en (a) pour produire un composé de la formule 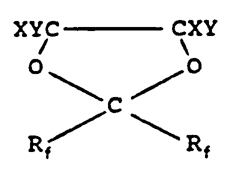 où X est Cl et Y est Cl ou F, au moins un Y étant F, et où chaque R_{f} est indépendamment un groupe perhalogénoalkyle dont l'atome de carbone alpha est substitué par au moins un atome de fluor,
le perfectionnement consistant à conduire la chloration de (a) en présence d'un catalyseur qui est au moins l'un de La, Ni, Sn, Zn, Fe, Co ou Cu.

4. Dans un procédé pour la production d'un 2,2-bis(perhalogénoalkyl)-1,3-dioxolanne halogéné consistant à
(a) chlorer un 2,2-bis(perhalogénoalkyl)-1,3-dioxolanne, et
(b) fluorer le composé produit en (a) pour produire un composé de la formule 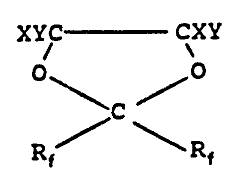 où X est Cl et Y est Cl ou F, au moins un Y étant F, et où chaque R_{f} est indépendamment un groupe perhalogénoalkyle dont l'atome de carbone alpha est substitué par au moins un atome de fluor,
le perfectionnement consistant à conduire la fluoration de (b) en présence d'un catalyseur qui est au moins l'un de Cr, La, Ni, Sn, Zn, Fe, Co ou Cu.

5. Un procédé selon la revendication 1, dans lequel le catalyseur est sous la forme d'un chlorure métallique supporté.

6. Un procédé selon la revendication 5, dans lequel le catalyseur est sous la forme de chlorure métallique supporté par du carbone.

7. Un procédé selon la revendication 2, dans lequel le catalyseur est sous la forme d'un chlorure métallique supporté.

8. Un procédé selon la revendication 2, dans lequel le catalyseur est Cr₂O₃.

9. Un procédé selon la revendication 3, dans lequel le catalyseur utilisé dans (b) est Cr₂O₃.

10. Un procédé selon la revendication 1, dans lequel le catalyseur est CuCl₂/C.

11. Un procédé selon la revendication 1, dans lequel la température est d'environ 250 à 300°C, le rapport du chlore au 2,2-bis(perhalogénoalkyl)-1,3-dioxolanne est d'environ 4:1 à 10:1, le temps de réaction est d'environ 1 à 120 secondes et la pression de réaction est d'environ 0,1 à 2 MPa.

12. Un procédé selon la revendication 1, dans lequel R_{f} est CF₃.

13. Un procédé selon la revendication 2, dans lequel R_{f} est CF₃.

14. Dans un procédé pour la production de 2,2-bis(perhalogénoalkyl)-1,3-dioxole de la formule 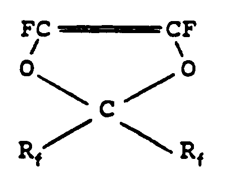 où chaque R_{f} est indépendamment un groupe perhalogénoalkyle dont l'atome de carbone alpha est substitué par au moins un atome de fluor, consistant à chlorer un 2,2-bis(perhalogénoalkyl)-1,3-dioxolanne correspondant, à fluorer le produit et à déshalogéner ensuite pour former le 1,3-dioxole,
le perfectionnement consistant à conduire la chloration en présence d'un catalyseur qui est au moins l'un de La, Ni, Sn, Zn, Fe, Co ou Cu.

15. Un procédé selon la revendication 14, dans lequel le perfectionnement consiste de plus à conduire la fluoration en présence d'un catalyseur qui est au moins l'un de Cr, La, Ni, Sn, Zn, Fe, Co ou Cu.

16. Un procédé selon la revendication 14, dans lequel le perfectionnement consiste de plus, avant la déshalogénation, à séparer le *trans*-2,2-bis(perhalogénoalkyl)-4,5-difluoro-4,5-dichloro-1,3-dioxolanne et à ne déshalogéner sensiblement que ledit isomère.

17. Un procédé selon la revendication 15, dans lequel le perfectionnement consiste de plus, avant la déshalogénation, à séparer le *trans*-2,2-bis(perhalogénoalkyl)-4,5-difluoro-4,5-dichloro-1,3-dioxolanne et à ne déshalogéner sensiblement que ledit isomère.

18. Un procédé pour la production d'un 2,2-bis(perhalogénoalkyl)-1,3-dioxole de la formule 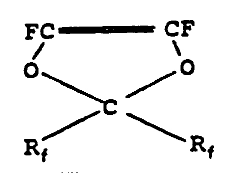 où chaque R_{f} est indépendamment un groupe perhalogénoalkyle dont l'atome de carbone alpha est substitué par au moins un atome de fluor, consistant à déshalogéner un *trans*-2,2-bis(perhalogénoalkyl)-4,5-difluoro-4,5-dichloro-1,3-dioxolanne correspondant qui a été séparé d'un mélange dudit isomère *trans* et d'autres isomères.

19. Dans un procédé pour la production de 2,2-bis(perhalogénoalkyl)-1,3-dioxole de la formule 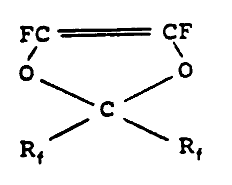 où chaque R_{f} est indépendamment un groupe perhalogénoalkyle dont l'atome de carbone alpha est substitué par au moins un atome de fluor, consistant à chlorer un 2,2-bis(perhalogénoalkyl)-1,3-dioxolanne correspondant, à fluorer le produit, à déshalogéner ensuite pour former le 1,3-dioxole,
le perfectionnement consistant à conduire la fluoration en présence d'un catalyseur qui est au moins l'un de Cr, La, Ni, Sn, Zn, Fe, Co ou Cu.

20. Dans un procédé pour la préparation d'un polymère formé à partir de monomères qui comprennent des 2,2-bis(perhalogénoalkyl)-1,3-dioxoles de la formule 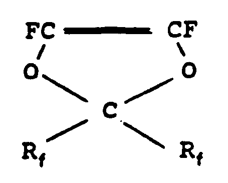 où chaque R_{f} est indépendamment un groupe perhalogénoalkyle dont l'atome de carbone alpha est substitué par au moins un atome de fluor, le perfectionnement consistant à préparer les dioxoles par déshalogénation des *trans*-2,2-bis(perhalogénoalkyl)-4,5-difluoro-4,5-dichloro-1,3-dioxolannes correspondants séparés d'un mélange d'isomères *cis* et *trans*.

21. Un procédé selon la revendication 20, dans lequel le polymère est produit par homopolymérisation du dioxole, copolymérisation du dioxole avec le tétrafluoréthylène ou copolymérisation du dioxole avec le fluorure de vinylidène.

22. Dans un procédé pour la préparation d'un polymère formé à partir de monomères qui comprennent des 2,2-bis(perhalogénoalkyl)-1,3-dioxoles de la formule 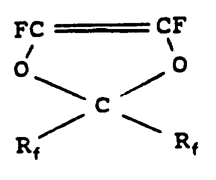 où chaque R_{f} est indépendamment un groupe perhalogénoalkyle dont l'atome de carbone alpha est substitué par au moins un atome de fluor, le perfectionnement consistant à préparer les dioxoles par déshalogénation des *trans*-2,2-bis(perhalogénoalkyl)-4,5-difluoro-4,5-dichloro-1,3-dioxolannes correspondants produits en augmentant la quantité d'isomère *trans* dans un mélange d'isomères *cis* et *trans* par transposition d'isomère *cis* en isomère *trans*.
